# FASCICULE DE BREVET EUROPEEN

(11) **EP 3 656 438 B1**
(45) Date de publication et mention de la délivrance du brevet: **23.06.2021**
(21) Numéro de dépôt: 19209437.3
(22) Date de dépôt: 15.11.2019
(51) Int. Cl.: A61N 1/05, A61N 1/375

(54) **SONDE MEDICALE IMPLANTABLE AVEC DISPOSITIF REDUCTEUR DE TENSION**
IMPLANTIERBARE MEDIZINISCHE ELEKTRODE MIT ZUGENTLASTUNG
IMPLANTABLE MEDICAL LEAD WITH STRAIN RELIEF ASSEMBLY

(30) Priorité: 21.11.2018 FR 1871665
(43) Date de publication de la demande: 27.05.2020
(73) Titulaire: Sorin CRM SAS, 92140 Clamart (FR)
(72) Inventeur: JULIEN, Etienne, 92310 Sèvres (FR); SHAN, Nicolas, 92160 Antony (FR); RAULT, Maxime, 75015 Paris (FR)
(74) Mandataire: Grünecker Patent- und Rechtsanwälte PartG mbB

(56) Documents cités:
- EP-A2- 1 426 079
- EP-A2- 2 571 569
- WO-A1-2017/191507
- US-A1- 2011 257 659
- US-A1- 2013 005 169
- US-A1- 2016 158 558
- US-B1- 7 680 544
- US-B2- 8 295 948
- US-B2- 8 417 343
- US-B2- 8 939 905

## Description

La présente invention se rapporte une sonde médicale implantable comprenant un dispositif réducteur de tension, ainsi qu'à un connecteur de sonde et à une méthode de fabrication d'une telle sonde implantable.

Les sondes médicales implantables sont communément utilisées en association avec des dispositifs médicaux implantables, tels que des dispositifs de stimulation cardiaque, de défibrillation ou/et de neuromodulation.

Afin, par exemple, de délivrer une stimulation électrique à un patient, une sonde implantable comprend une ou plusieurs électrodes qui sont implantées dans le cœur du patient. Des fils conducteurs logés à l'intérieur de la sonde implantable permettent de coupler électriquement la ou les électrodes à un connecteur de la sonde qui est lui-même couplé au dispositif médical implantable. Ainsi, le dispositif peut détecter une activité électrique cardiaque et/ou délivrer une thérapie électrique adaptée au moyen du ou des électrodes de la sonde électriquement connectées au dispositif.

Le raccordement électrique entre les fils conducteurs et le connecteur de la sonde, réalisé par soudure par exemple, est susceptible d'être sollicité mécaniquement, notamment lors de l'implantation de la sonde, puis au cours de la vie du patient. Il est alors nécessaire que les fils conducteurs, en particulier ceux dont le diamètre est inférieur à 200 micromètres, puissent supporter de telles contraintes mécaniques, notamment en tension, afin d'éviter une rupture au niveau de la connexion électrique avec le connecteur ou du fil conducteur lui-même.

Il est connu du document US 2013/0005169 A1 de placer un tube flexible en matériau biocompatible autour du corps de sonde implantable afin de réduire les contraintes en tension au niveau de l'extrémité distale de la sonde. Le document US 2011/0257659 A1 décrit aussi un dispositif réducteur de tension en forme de tube flexible dans lequel est introduite la sonde. Cependant, ces dispositifs réducteurs de tension sont des éléments supplémentaires à la sonde qui sont mis en contact direct avec les tissus cardiaques, ce qu'il est préférable d'éviter afin de réduire le risque de contamination.

Le document US 7,680,544 B1, quant à lui, décrit une sonde comprenant un dispositif réducteur de contrainte logé dans un segment de la sonde en dehors du boîtier du dispositif médical implantable. Ce dispositif comprend une structure dont des éléments (85a, 85b, 85c) s'étendent radialement par rapport au corps tubulaire de la sonde tel que des câbles conducteurs sont enroulés manière hélicoïdale autour du corps tubulaire de la sonde. De ce fait, en plus d'occuper davantage d'espace afin de répartir les éléments (85a, 85b, 85c) qui s'étendent radialement, la sonde décrite par US 7,680,544 B1 n'est pas adaptée pour absorber des contraintes fortes qui sont transmises à la soudure entre le connecteur et les fils.

L'objet de la présente invention est ainsi d'améliorer la résistance en tension des fils conducteurs d'une sonde implantable, afin de sécuriser et de maintenir la connexion électrique des fils conducteurs au connecteur de la sonde.

L'objet de la présente invention est atteint avec une sonde implantable comprenant au moins un fil conducteur et un connecteur configuré pour être connecté à un dispositif médical implantable de type dispositif de stimulation cardiaque, de défibrillation ou/et de neuromodulation, et comprenant en outre un dispositif réducteur de tension qui s'étend longitudinalement selon un axe à partir d'une extrémité du connecteur et essentiellement parallèle à l'axe longitudinal du connecteur et qui comprend au moins un élément avec une paroi de déviation ; tel que le fil conducteur est dévié par la paroi de déviation du dispositif réducteur de tension de manière à ce que l'élément de déviation du dispositif réducteur de tension bloque le fil conducteur contre la paroi de déviation lorsque le fil conducteur est sollicité en tension; et la surface de déviation s'étend selon un axe qui est désaligné avec l'axe longitudinal du dispositif réducteur de tension. L'agencement du fil conducteur autour du dispositif réducteur de tension permet de dévier d'une manière non-radiale le fil conducteur autour de l'axe longitudinal du dispositif réducteur de tension au moyen de l'élément de déviation tel que le fil conducteur se bloque sur la paroi de déviation lorsque le fil conducteur est sollicité en tension. L'avantage du blocage du fil conducteur sur la paroi de déviation est qu'il permet d'éviter que toute la longueur du fil conducteur soit axialement sollicitée en tension. De ce fait, l'agencement du fil conducteur autour du dispositif permet de réduire la tension subie par le fil conducteur. De plus, la déviation du fil conducteur par la paroi de déviation, dont l'axe est désaligné avec l'axe longitudinal du dispositif réducteur de tension, permet de raccourcir la distance nécessaire pour agencer le fil conducteur autour du dispositif réducteur de tension, en particulier en comparaison avec des dispositifs tubulaires autour duquel le fil conducteur est enroulé radialement, c'est-à-dire de manière hélicoïdale autour de l'axe longitudinale du dispositif. En conséquence, la déviation du fil conducteur par l'élément de déviation du dispositif réducteur de tension permet de réduire les sollicitations mécaniques subies par le fil conducteur en tension et donc de diminuer le risque de rupture du fil conducteur lui-même et au niveau de la connexion électrique avec le connecteur de sonde. De ce fait, le dispositif réducteur de tension permet d'améliorer la fiabilité et la longévité de la connexion électrique entre le fil conducteur et le connecteur de la sonde.

La sonde implantable selon la présente invention peut être davantage améliorée grâce aux modes de réalisation suivants.

Selon une autre réalisation de l'invention, l'élément déviation peut comprendre un trou traversant ou/et une encoche dont la profondeur s'étend selon un axe désaligné avec l'axe longitudinal du dispositif réducteur de tension; ou/et une protubérance qui fait saillie depuis le dispositif réducteur de tension selon un axe (P, P') désaligné avec l'axe longitudinal du dispositif réducteur de tension. Le dispositif réducteur de tension comprend ainsi un ou plusieurs éléments de déviation permettant de dévier le fil conducteur, c'est-à-dire d'empêcher au moins partiellement un enroulement hélicoïdal du fil conducteur autour de l'axe longitudinal du dispositif, ce qui permet de réduire la longueur du dispositif nécessaire à la réduction en tension.

Selon une autre réalisation de l'invention, au moins une section du fil conducteur peut être agencée en boucle autour de la paroi de déviation d'une protubérance qui fait saillie depuis une surface extérieure du dispositif réducteur de tension. Ainsi, la protubérance fournit un moyen d'arrêt au fil conducteur lorsque que celui-ci est agencé en boucle autour de la paroi de la protubérance.

Selon une autre réalisation de l'invention, la protubérance peut avoir une forme cylindrique et un axe de révolution désaligné avec l'axe longitudinal du dispositif réducteur de tension. La structure cylindrique de la protubérance facilite l'agencement du fil conducteur autour de la protubérance de par sa section transversale circulaire et évite la présence de bord ou d'arêtes coupants qui pourraient abîmer le fil conducteur, en particulier lorsque qu'il se bloque sur la paroi de la protubérance en réponse à une sollicitation en tension.

Selon une autre réalisation de l'invention, le fil conducteur peut être introduit au moins deux fois à travers le trou traversant de manière à ce que le fil conducteur est agencé en boucle autour d'une portion du dispositif réducteur de tension. Ainsi, le trou traversant fournit un moyen d'arrêt au fil conducteur lorsque que celui-ci est agencé en boucle autour des parois du trou traversant.

Selon une autre réalisation de l'invention, la paroi de déviation peut s'étendre selon un axe qui est désaligné avec l'axe longitudinal du dispositif réducteur de tension de manière à former un angle essentiellement droit, en particulier un angle droit à plus ou moins 10 degrés. De ce fait, la paroi de déviation dévie le fil conducteur afin que le fil conducteur ne soit pas agencé de manière radiale autour de l'axe longitudinale du dispositif réducteur de tension au moins sur une portion du dispositif réducteur de tension. L'agencement non-radial du fil conducteur permet d'améliorer la retenue du fil conducteur au dispositif réducteur de tension et d'augmenter la capacité du fil conducteur à absorber des contraintes en tension

Selon une autre réalisation de l'invention, au moins deux sections du fil conducteur peuvent se chevaucher et/ou se superposer. Ainsi, les frottements entre des sections du fil conducteur et avec le dispositif réducteur de tension sont augmentés ce qui contribue à éviter qu'une force de tension appliquée au fil ne puisse défaire l'agencement du fil conducteur autour du dispositif réducteur de tension.

Selon une autre réalisation de l'invention, le connecteur et le dispositif de réducteur de tension peuvent chacun être pourvus d'un ou de plusieurs lumens ; au moins un lumen du dispositif de réducteur de tension étant aligné avec un lumen du connecteur. De ce fait, le dispositif réducteur de tension est adapté pour une sonde implantable dont le connecteur est pourvu d'un ou plusieurs lumens.

Selon une autre réalisation de l'invention, la sonde implantable peut comprendre plusieurs fils conducteurs tel que chaque fil conducteur est dévié par le dispositif réducteur de tension de manière à ce que l'élément de déviation du dispositif réducteur de tension bloque les fils conducteurs lorsque les fils conducteurs sont sollicités en tension.

Selon une autre réalisation de l'invention, les fils conducteurs peuvent sortir d'une extrémité du connecteur de manière distincte les uns des autres et sont regroupés en un fil multi-brin par leur agencement autour du dispositif réducteur de tension. De ce fait, en plus d'améliorer la résistance en tension des fils conducteurs par leur déviation causée par la paroi de déviation, le dispositif réducteur de tension permet aussi de simplifier l'agencement des fils conducteurs en les réunissant dans un seul fil multi-brins.

Selon une autre réalisation de l'invention, le dispositif réducteur de tension peut être attaché à une extrémité du connecteur par collage, par emboîtement, par raccordement encliquetable ou/et par un ou des éléments de fixation. Le dispositif réducteur de tension est ainsi facilement attachable au connecteur.

Alternativement, le dispositif réducteur de tension et le au moins un élément de déviation peuvent être formés de manière intégrale avec le connecteur. De la sorte, le nombre de composant est réduit, ce qui permet de diminuer les coûts et évite une étape de montage pour la fixation du dispositif réducteur de tension au connecteur.

L'objet de la présente invention est aussi atteint avec un dispositif médical implantable de type dispositif de stimulation cardiaque, de défibrillation ou/et de neuromodulation comprenant une sonde implantable, elle-même comprenant un connecteur, un dispositif réducteur de tension d'axe longitudinal et au moins un fil conducteur connecté électriquement au connecteur ; tel que le dispositif réducteur de tension s'étend longitudinalement à partir d'une extrémité du connecteur selon un axe et qui est essentiellement parallèle à l'axe longitudinal du connecteur, et comprend au moins un élément avec une paroi de déviation; le connecteur et le dispositif réducteur de tension étant configurés pour être logés dans le dispositif médical implantable; de sorte que le fil conducteur est dévié par la paroi de déviation du dispositif réducteur de tension de manière à ce que la paroi de déviation du dispositif réducteur de tension bloque le fil conducteur lorsque le fil conducteur est sollicité en tension ; et l'élément la paroi de déviation s'étend selon un axe qui est désaligné avec l'axe longitudinal du dispositif réducteur de tension. L'avantage du blocage du fil conducteur sur l'élément de déviation est qu'il permet d'éviter que toute la longueur du fil conducteur soit axialement sollicitée en tension. De ce fait, l'agencement du fil conducteur autour du dispositif permet de réduire la tension subie par le fil conducteur. De plus, la déviation du fil conducteur par la paroi de déviation permet de dévier d'une manière non-radiale le fil conducteur autour de l'axe longitudinal du dispositif réducteur de tension. Cette configuration, en comparaison avec des dispositifs tubulaires autour duquel le fil conducteur est enroulé radialement, c'est-à-dire de manière hélicoïdale autour de l'axe longitudinale du dispositif, permet d'améliorer la mise en place et la tenue du fil conducteur autour du dispositif réducteur de tension et d'améliorer sa capacité à absorber des contraintes en tension. Enfin, le fait que le dispositif réducteur de tension soit positionné à l'intérieur du dispositif médical implantable, cela permet d'éviter que le dispositif réducteur de tension, une fois implanté, ne soit en contact direct avec les tissus du patient, et donc de limiter le risque de contamination - notamment du sang.

De plus, l'objet de la présente invention est également atteint avec une méthode de fabrication d'une sonde implantable configurée pour être connecté au moyen d'un fil ou de plusieurs fils conducteurs et d'un connecteur à un dispositif médical implantable de type dispositif de stimulation cardiaque, de défibrillation ou/et de neuromodulation, la sonde implantable comprenant en outre un dispositif réducteur de tension d'axe longitudinal pourvu à une extrémité du connecteur, le dispositif réducteur de tension comprenant lui-même au moins un élément avec une paroi de déviation ; ladite méthode comprenant une étape de déviation du ou des fils conducteurs par le dispositif réducteur de tension de manière à agencer le ou les fils conducteurs du connecteur autour de la paroi de déviation qui s'étend selon un axe qui est désaligné avec l'axe longitudinal du dispositif réducteur de tension, de manière à ce que lorsque le ou les fils conducteurs sont sollicités en tension, le ou les fils conducteurs se bloquent contre la paroi de déviation du dispositif réducteur de tension .Cette méthode permet de réduire les sollicitations mécaniques subies par le fil conducteur en tension car le fait d'agencer et de dévier le fil conducteur autour du dispositif réducteur de tension permet au fil conducteur de se bloquer sur la paroi de déviation lorsqu'il est sollicité en tension. En conséquence, l'agencement et la déviation du fil conducteur autour du dispositif réducteur de tension permet de diminuer le risque de rupture du fil conducteur lui-même et au niveau de la connexion électrique avec le connecteur de sonde. De ce fait, le dispositif réducteur de tension permet aussi d'améliorer la fiabilité et la longévité de la connexion électrique entre le fil conducteur et le connecteur de la sonde. En effet, cette configuration, en comparaison avec des dispositifs tubulaires autour duquel le fil conducteur est enroulé radialement, c'est-à-dire de manière hélicoïdale autour de l'axe longitudinale du dispositif, permet d'améliorer la mise en place et la tenue du fil conducteur autour du dispositif réducteur de tension et d'améliorer sa capacité à absorber des contraintes en tension.

Les modes de réalisation peuvent être combinés pour former davantage de variantes de mode de réalisation avantageux de la présente invention.

L'invention et ses avantages seront expliqués plus en détail dans la suite au moyen de modes de réalisation préférés et en s'appuyant notamment sur les figures d'accompagnement suivantes, dans lesquelles :
Figure 1 représente schématiquement un dispositif médical implantable pourvu d'une sonde implantable.
Figure 2 représente un connecteur et un dispositif réducteur de tension selon un premier mode de réalisation de la présente invention.
Figure 3 représente une portion du connecteur et un dispositif réducteur de tension selon un troisième mode de réalisation de la présente invention.
Figure 4 représente une portion du connecteur et un dispositif réducteur de tension selon un quatrième mode de réalisation de la présente invention.
Figure 5 représente une portion du connecteur et un dispositif réducteur de tension selon un cinquième mode de réalisation de la présente invention.
Figure 6 représente une portion du connecteur et un dispositif réducteur de tension selon un sixième mode de réalisation de la présente invention.
Figure 7 représente une portion du connecteur et un dispositif réducteur de tension selon un septième mode de réalisation de la présente invention.
Figure 8 représente une portion du connecteur et un dispositif réducteur de tension selon un huitième mode de réalisation de la présente invention.
Figure 9 représente une portion du connecteur et un dispositif réducteur de tension selon un neuvième mode de réalisation de la présente invention.

L'invention va maintenant être décrite plus en détail en utilisant des modes de réalisation avantageux d'une manière exemplaire et en référence aux dessins. Les modes de réalisation décrits sont simplement des configurations possibles et il faut garder à l'esprit que les caractéristiques individuelles telles que décrites ci-dessus peuvent être fournies indépendamment les unes des autres ou peuvent être omises tout à fait lors de la mise en oeuvre de la présente invention.

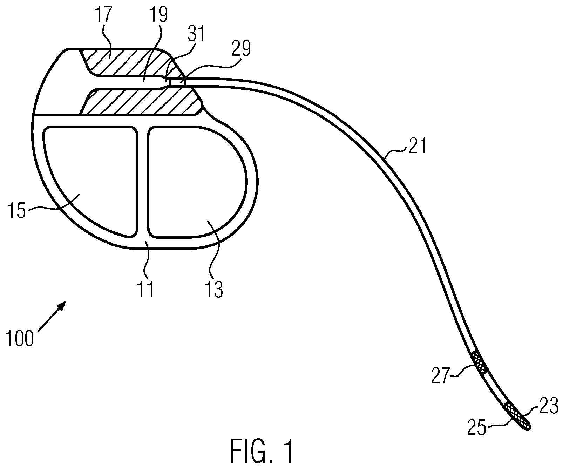

La **Figure 1** illustre schématiquement un dispositif médical implantable 100 de type dispositif de stimulation cardiaque, de défibrillation ou/et de neuromodulation. Le dispositif médical implantable 100 est pourvu d'un boîtier métallique 11 qui comprend un circuit électrique 13 et une pile 15, en particulier une pile lithium. Le dispositif médical implantable 100 comprend également un bloc de connexion 17 en matière plastique dans lequel est logé un connecteur 19 de sonde implantable 21. Le connecteur 19 peut être réalisé à partir de matériaux conducteurs biocompatibles tels que l'acier inoxydable 316L ou un alliage métallique, MP35N, par exemple. Le matériau du connecteur 19 peut être sélectionné pour être biocompatible ainsi que pour conduire et transmettre de manière appropriée des signaux électriques provenant du dispositif de stimulation électrique 100. En effet, le connecteur 19 sert de point de raccordement entre le dispositif médical implantable 100 et la sonde implantable 21. La sonde implantable 21, dont une extrémité distale 23 est pourvue d'une ou plusieurs d'électrodes et/ou capteurs 25, 27 aptes être implantées dans le cœur du patient, comprend des fils conducteurs (non-représentés sur la Figure 1) logés à l'intérieur de la sonde implantable 21. Ces fils conducteurs permettent de coupler électriquement les électrodes et/ou capteurs 25, 27 au connecteur 19 de la sonde 21 qui est lui-même couplé au dispositif médical implantable 100. Ainsi, le dispositif 100 peut détecter une activité électrique cardiaque et/ou délivrer une thérapie électrique adaptée au moyen des électrodes et/ou capteurs 25, 27 de la sonde 21.

La sonde implantable 21 est donc connectée électriquement au dispositif médical implantable 100 au moyen d'un raccordement entre les fils conducteurs de la sonde 21 et le connecteur 19, réalisé par soudure par exemple. Afin de protéger et maintenir ce raccordement électrique, notamment lorsque les fils conducteurs de la sonde 21 sont sollicités en tension lors de l'implantation du dispositif 100 et au cours de la vie du patient, un dispositif réducteur de tension 29 est attaché à une extrémité proximale 31 du connecteur 19 logé dans le bloc de connexion 17. Ainsi, le dispositif réducteur de tension 29 est positionné à l'intérieur du dispositif médical implantable 100, ce qui permet d'éviter que le dispositif réducteur de tension 29 ne soit en contact direct avec les tissus du patient, et donc de limiter le risque de contamination - notamment du sang. De plus, le fait que le dispositif réducteur de tension 29 soit logé dans le bloc de connexion 17 avec le connecteur 19 permet de réduire le nombre de manipulation du chirurgien lors de l'implantation du dispositif 100 dans le corps d'un patient ou du remplacement de la sonde 21, en comparaison avec des dispositifs réducteurs de tension connus de l'état de l'art et qui sont disposés autour de la sonde à l'extérieur du bloc de connexion et du connecteur.

Le dispositif réducteur de tension 29 sera à présent davantage décrit dans ce qui suit selon plusieurs modes de réalisation de la présente invention. Ainsi, chacun des dispositifs réducteurs de tension 200, 300, 400, 500, 600, 700, 800, 900 qui sera décrit ci-dessous est configuré pour la sonde implantable 21 décrite à la Figure 1.

Les éléments, en particulier relatifs au connecteur de sonde, avec les mêmes références numériques déjà utilisées pour la description de la Figure 1 ne seront pas décrits à nouveau en détail, et référence est faite à leurs descriptions ci-dessus.

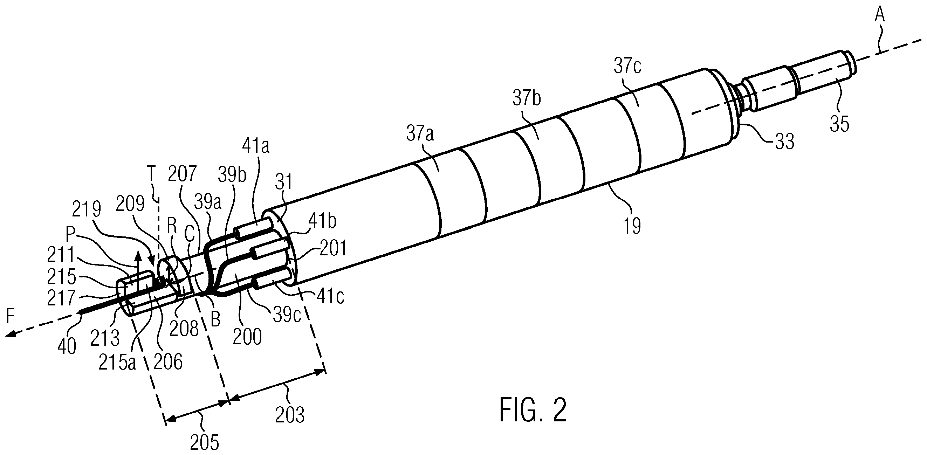

La **Figure 2** illustre le connecteur 19 et un dispositif réducteur de tension 200 selon un premier mode de réalisation de la présente invention.

Le connecteur 19 a une forme cylindrique d'axe A et comprend au niveau de son extrémité distale 33 une broche distale 35 configurée pour être connectée au bloc de connexion 17 du dispositif médical implantable 100 représenté à la Figure 1.

Le connecteur 19 représenté à la Figure 2 est de type multipolaire car il est pourvu de trois contacts 37a, 37b, 37c. Dans une variante, le connecteur 19 peut comprendre plus ou moins que trois contacts. Dans une autre variante, le connecteur 19 peut comprendre un seul contact.

La sonde implantable 21 comprend des fils conducteurs 39a, 39b, 39c, électriquement isolés des uns des autres, qui permettent de connecter électriquement les contacts 37a, 37b, 37c du connecteur 19 aux électrodes 25, 27 (voir Figure 1) de la sonde 21. Les fils conducteurs 39a, 39b, 39c sont ainsi chacun logés et soudés à un hypotube métallique respectif 41a, 41b, 41c du connecteur 19 au niveau de l'extrémité proximale 31 du connecteur 19. Dans une variante, la sonde 21 peut comprendre plus ou moins que trois fils conducteurs. Dans une autre variante, plusieurs fils conducteurs peuvent être logés et soudés à un même hypotube du connecteur.

Un risque de rupture du raccordement électrique entre les fils conducteurs 39a, 39b, 39c et le connecteur 19 de la sonde 21, réalisé par soudure dans le mode de réalisation illustré par la Figure 2, peut être réduit par le biais du dispositif réducteur de tension 200 selon l'invention.

Selon le premier mode de réalisation de l'invention, le dispositif réducteur de tension 200 a une forme allongée et une extrémité 201 qui est attachée à l'extrémité proximale 31 du connecteur 19. Par exemple, le dispositif réducteur de tension 200 peut être attaché à l'extrémité proximale 31 du connecteur 19 par collage, par emboîtement, par raccordement encliquetable ou/et par un ou des éléments de fixation.

Dans une variante, le dispositif réducteur de tension 200 est formé de manière intégrale avec le connecteur 19 afin de réduire le nombre de pièces détachées à assembler et de faciliter le montage du connecteur 19.

Le dispositif réducteur de tension 200 comprend une première portion 203 pourvue de l'extrémité 201 attachée au connecteur 19. La portion 203 a une forme cylindrique de section transversale circulaire telle que la section transversale en tout point de la portion 203 est plus petite que la section transversale en tout point du connecteur 19. De cette manière, le dispositif réducteur de tension 200 a une dimension transversale adaptée pour pouvoir être attaché à l'extrémité proximale 31 du connecteur 19 entre les hypotubes 41a, 41b, 41c qui font eux aussi saillie depuis l'extrémité proximale 31 du connecteur 19.

Le dispositif réducteur de tension 200 comprend une seconde portion 205 comprenant une forme de semi-cylindre à section pleine 206 coupé selon son axe de révolution B qui est aligné avec l'axe longitudinal A du connecteur 19.

La seconde portion 205 est pourvue d'une encoche 219 réalisée entre un premier moyen d'arrêt 209 et un second moyen d'arrêt 211, dont la profondeur T de l'encoche 219 s'étend de manière perpendiculaire à l'axe de révolution B du semi-cylindre 206. Ainsi, l'axe qui s'étend le long de la profondeur T de l'encoche 219 et l'axe de révolution B du dispositif réducteur de tension sont désalignés l'un par rapport à l'autre. Selon le premier mode de réalisation, l'encoche 219 constitue un élément de déviation du dispositif réducteur de tension 200 de manière à ce que cet élément de déviation 219 s'étend selon l'axe T qui est désaligné avec l'axe longitudinal B du dispositif réducteur de tension 200.

Le premier moyen d'arrêt 209, ici en forme de demi-disque 208 dont le rayon R est plus grand que celui de la première portion cylindrique 203 et dont le centre C est positionné sur l'axe de révolution B. Le premier moyen d'arrêt 209 est positionné au niveau d'une jonction 207 entre la première 203 et la deuxième portion 205 du dispositif réducteur de tension 200 de manière à ce que le diamètre de la section plane (non visible sur la Figure 2) du demi-disque 208 soit essentiellement perpendiculaire à l'axe de révolution B du semi-cylindre 206.

Le second moyen d'arrêt 211 fait saillie depuis la surface plane 213 du semi-cylindre 106 selon une direction P qui est perpendiculaire à l'axe B du dispositif réducteur de tension 200. Ici, le second moyen d'arrêt 211 a une paroi de déviation 215. L'angle 215a de la paroi de déviation 215 du second moyen d'arrêt 211 du côté de l'encoche 219 est arrondi de manière à ne pas endommager les fils conducteurs 39a, 39b, 39c lorsqu'ils sont bloqués contre la paroi de déviation 215 sous l'effet d'une sollicitation en tension d'une force F.

Pour se faire, les fils conducteurs 39a, 39b, 39c sortent de leurs hypotubes respectifs 41a, 41b, 41c, auxquels ils sont soudés, au niveau de l'extrémité proximale 31 du connecteur 19 et sont insérés dans l'encoche 219 puis déviés par la paroi de déviation 215a du moyen d'arrêt 211, qui constitue un élément de déviation du dispositif réducteur de tension 200.

L'agencement des fils conducteurs 39a, 39b, 39c par rapport à l'encoche 219 et aux premier et deuxième moyens d'arrêt 209, 211 du dispositif réducteur de tension 200 permet aux fils conducteurs 39a, 39b, 39c de pouvoir être bloqués contre la paroi de déviation 215 lorsqu'ils sont sollicités en tension, c'est-à-dire que lorsque les fils conducteurs 39a, 39b, 39c sont sollicités en tension par une force représentée par une flèche F, ils se bloquent sur le dispositif réducteur de tension 200 au niveau de la paroi de déviation 215. Cela permet notamment de réduire les sollicitations mécaniques subies par les fils conducteurs 39a, 39b, 39c en tension et donc de diminuer le risque de rupture des fils conducteurs 39a, 39b, 39c eux-mêmes ainsi qu'au niveau de la connexion électrique avec les hypotubes 41a, 41b, 41c du connecteur 19 de sonde 21.

Selon un deuxième mode de réalisation, non-représenté à la Figure 2, les fils conducteurs 39a, 39b, 39c sont insérés à travers l'encoche 219 puis enroulés au moins une fois autour du second moyen d'arrêt 211 afin d'éviter un glissement involontaire des fils conducteurs 39a, 39b, 39c hors de la surface 213 du semi-cylindre 206.

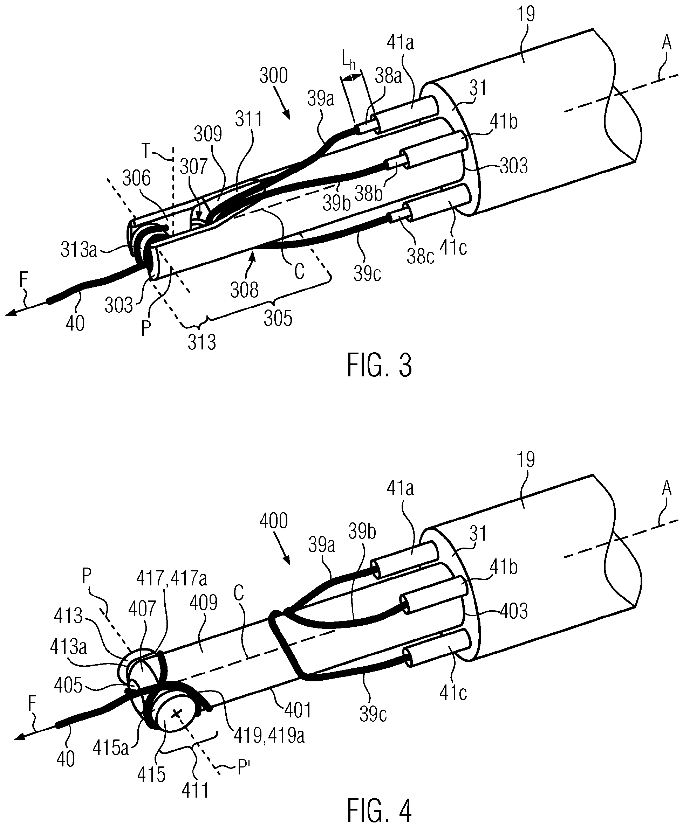

La **Figure 3** illustre une portion du connecteur 19 et un dispositif réducteur de tension 300 selon un troisième mode de réalisation de la présente invention. Les éléments, en particulier relatifs au connecteur de sonde, avec les mêmes références numériques déjà utilisées pour la description des Figures 1 et 2 ne seront pas décrits à nouveau en détail, et référence est faite à leurs descriptions ci-dessus.

Le dispositif réducteur de tension 300 de la Figure 3 selon le troisième mode de réalisation a une forme cylindrique allongée d'axe C et une extrémité 301 qui est attachée à l'extrémité proximale 31 du connecteur 19 ainsi qu'une deuxième extrémité 303, opposée à l'extrémité 301.

Selon le troisième mode de réalisation, le fil conducteur 39a est logé et soudé à un hypotube intermédiaire 38a et l'hypotube intermédiaire 38a est lui-même logé et soudé dans l'hypotube 41a du connecteur 19 au niveau de l'extrémité proximale 31 du connecteur 19.

Une soudure au laser du fil conducteur 39a à l'hypotube intermédiaire 38a permet de connecter électriquement ce dernier au fil conducteur 39a. L'hypotube intermédiaire 38a est logé et soudé par soudure laser avec l'hypotube correspondant 41a de manière à ce qu'une portion de longueur lₕ de l'hypotube intermédiaire 38a fait saillie hors de l'hypotube 41a et tel que l'hypotube intermédiaire 38a est électriquement connecté avec l'hypotube 41a. Cette soudure entre l'hypotube intermédiaire 38a et l'hypotube 41a est réalisée en dehors du connecteur 19 ce qui permet de faciliter la réalisation de la soudure entre ces deux hypotubes 38a, 41a en offrant davantage d'espace et de visibilité à un opérateur pour effectuer la soudure au laser.

La soudure entre le fil conducteur 39a et l'hypotube intermédiaire 39a, ainsi que la soudure des hypotubes 39a, 41a, sont réalisées par soudure laser. La soudure au laser permet notamment de réaliser une soudure étanche, et permet également une soudure précise particulièrement adaptée à l'échelle de connecteurs de sondes implantables de dispositifs médicaux.

Ainsi, l'hypotube intermédiaire 38a sert d'intermédiaire à la connexion électrique entre le fil conducteur 39a et l'hypotube 41a du connecteur 19. L'utilisation de l'hypotube intermédiaire 38a rend alors possible une connexion électrique entre un connecteur 19 comprenant un hypotube 41a, 41b, 41c aux dimensions standards comprises entre 350 et 500 micromètres et un fil conducteur 31a, 39b, 39c de diamètre inférieur à 150 micromètres.

Le nombre d'hypotubes, le nombre d'hypotubes intermédiaires et le nombre de fils conducteurs ne sont pas limités à l'illustration de la Figure 3. Ainsi, chaque hypotube 41a, 41b, 41c pourrait chacun comprend un hypotube intermédiaire dans lequel chaque fil conducteur 39a, 39b, 39c y serait logé, soudé et connecté électriquement. Dans une variante, plusieurs fils conducteurs peuvent être logés dans un même hypotube intermédiaire.

Une portion 305 du dispositif réducteur de tension 300 vers l'extrémité libre 303 comprend un premier élément de déviation, ici un trou traversant 307 dont la profondeur s'étend selon un axe T. De préférence, le trou 307 pénètre à travers la portion 305 de forme cylindrique allongée tel qu'il y a intersection entre l'axe T du trou traversant 307 et l'axe C de la forme cylindrique allongée. De ce fait, l'axe T et l'axe C sont désalignés entre eux. Le trou traversant 307 est dimensionné de manière à ce que les fils conducteurs 39a, 39b, 39c puissent être insérés de part et d'autre du trou 307 tel que montré sur la Figure 3, ou tous insérés du même côté dans le trou traversant 307. De plus, le pourtour intérieur 309 du trou traversant 307 orienté du côté de l'extrémité 301 du dispositif réducteur de tension 300 est chanfreiné ou arrondi de manière à créer une pente 311 qui est décroissante vers le trou 307. La transition douce fournit par la pente 311 au niveau de la zone où sont introduits les fils conducteurs 39a, 39b, 39c dans le trou 307 permet de réduire le risque d'endommager les fils conducteurs 39a, 39b, 39c. Sur la Figure 3 seule une entrée 306 du trou traversant 307 est visible, celle comprenant le pourtour chanfreiné 309. L'autre entrée 308, ou sortie 308, opposée à l'entrée 306 du trou traversant 307, est également pourvue d'un pourtour chanfreiné ou arrondi.

Le trou traversant 307 est délimité du côté de l'extrémité libre 303 du dispositif réducteur de tension 300 par une portion 313. Ainsi, la portion 313 est comprise entre l'extrémité 303 et le trou traversant 307. Cette portion 313 est configurée pour que les fils conducteurs 39a, 39b, 39c, ayant été introduits dans le trou traversant 307, soient enroulés autour de la portion 313. La portion 313 est essentiellement cylindrique d'axe de révolution P perpendiculaire à l'axe C dispositif réducteur de tension 300. La paroi 313a de la portion 313 est une paroi de déviation du dispositif réducteur de tension 300.

Selon le troisième mode de réalisation, les fils conducteurs 39a, 39b sont insérés par l'entrée 306 dans le trou traversant 307 et ressortent par l'autre entrée 308, puis sont enroulés au moins une fois autour de la paroi de déviation 313a de la portion 313 selon l'axe P. Le fil conducteur 39c, quant à lui, est inséré par l'entrée 308 dans le trou traversant 307 et ressort par l'entrée 306, puis est enroulé au moins une fois dans le sens contraire des fils conducteurs 39a, 39b autour de la paroi de déviation 313a de la portion 313 selon l'axe P. Selon une variante, les fils conducteurs 39a, 39b, 39c peuvent être enroulés plusieurs fois autour de la paroi de déviation 313a de la portion 313. Ainsi, les fils conducteurs 39a, 39b, 39c sont regroupés en un fil multi-brin 40 au niveau de l'extrémité 303 du dispositif réducteur de tension 300.

De ce fait, les fils conducteurs 39a, 39b, 39c sont déviés par le trou traversant 307 qui forme une portion 313 dont la paroi de déviation 313a bloque les fils conducteurs 39a, 39b, 39c lorsqu'ils sont sollicités en tension. Les axes respectifs T, P des éléments de déviation 307, 313 s'étendent perpendiculairement par rapport à l'axe longitudinal C du dispositif réducteur de tension 300.

De ce fait, si le fil multi-brin 40 est tiré par une force de tension F, lors de l'implantation de la sonde par exemple, les fils conducteurs 39a, 39b, 39c se bloquent contre la paroi de déviation 313a du dispositif réducteur de tension 300 ce qui permet de les protéger de la sollicitation en tension. Cela permet aussi d'éviter que les fils conducteurs 39a, 39b, 39c ne soient tirés au niveau du raccordement avec les hypotubes intermédiaires 38a, 38b, 38c et les hypotubes 41a, 41b, 41c, ce qui pourrait casser la soudure entre les fils conducteurs et les hypotubes et donc endommager la connexion électrique.

Selon une variante, tous les fils conducteurs 39a, 39b, 39c peuvent traverser le trou 307 dans le même sens, soit par l'entrée 306 ou bien par l'entrée 308.

La **Figure 4** illustre une portion du connecteur 19 et un dispositif réducteur de tension 400 selon un quatrième mode de réalisation de la présente invention. Les éléments, en particulier relatifs au connecteur de sonde, avec les mêmes références numériques déjà utilisées pour la description des Figures 1 à 3 ne seront pas décrits à nouveau en détail, et référence est faite à leurs descriptions ci-dessus.

Le dispositif réducteur de tension 400 de la Figure 4 selon le quatrième mode de réalisation a une forme de cylindre circulaire droit 401 d'axe de révolution C. Une première extrémité 403 du cylindre 401 est attachée à l'extrémité proximale 31 du connecteur 19. Une seconde extrémité 405 du cylindre 401, opposée à l'extrémité 403, est représentée comme une extrémité libre 405 sur la Figure 4. L'axe de révolution C du dispositif réducteur de tension 400 est aligné avec l'axe A du connecteur 19. La section transversale de cylindre 401 est plus petite en tout point que la section transversale en tout point du connecteur 19.

L'extrémité libre 405 du dispositif réducteur de tension 400 est chanfreinée ou arrondie de manière à former une surface oblique 407 entre l'extrémité 405 et une paroi latérale 409 du cylindre 401. Cette surface chanfreinée 407 permet d'éviter une arête vive entre l'extrémité 405 et la paroi latérale 409 du cylindre 401. Ainsi, le chanfrein 407 permet de réduire le caractère potentiellement tranchant de l'arête entre l'extrémité 405 et la paroi latérale 409 du cylindre 401 qui pourrait endommager ou rompre les fils conducteurs 39a, 39b, 39c.

La paroi latérale 409 du cylindre 401 au niveau de l'extrémité 405 du dispositif réducteur de tension 400 est pourvue de deux éléments de déviations qui sont des protubérances 413, 415 de forme cylindrique et d'axes de révolution respectifs P et P'. Les protubérances 413, 415 sont en particulier positionnées tel que leurs axes de révolution P et P' sont alignés entre eux et tel que les axes de révolution P et P' intersectent l'axe C du cylindre 401 en un même point. Ainsi, les axes de révolution P, P' des éléments de déviations 413, 415 sont désalignés avec l'axe longitudinal C du cylindre 401 du dispositif 400. Chaque protubérance 413, 415 a une jonction 417, 419 avec la paroi latérale 409 du cylindre 401. Chaque jonction 417, 419 comprend un congés 417a, 419a afin d'arrondir la transition entre la paroi latérale 409 et les parois latérales respectives 413a, 415a des protubérances 413, 415. Les congés 417a, 417a permettent ainsi de réduire la concentration de contrainte que créerait un angle vif et ainsi réduit le risque d'endommager les fils conducteurs. Dans une variante, la paroi latérale 409 du cylindre 401 pourrait ne comprendre qu'une seule protubérance 413. Ainsi, le chanfrein 407 et les congés 417a, 419a du dispositif réducteur de tension 400 sont autant de caractéristiques structurelles qui permettent de réduire le risque d'endommager les fils conducteurs 39a, 39b, 39c.

Selon le quatrième mode de réalisation de l'invention, les fils conducteurs 39a, 39b, 39c sortent de leurs hypotubes respectifs 41a, 41b, 41c, auxquels ils sont soudés, au niveau de l'extrémité proximale 31 du connecteur 19. Afin de dévier les fils conducteurs 39a, 39b, 39c, les fils conducteurs 39a, 39b, 39c forment une boucle autour de la paroi latérale 413a de la protubérance 413 au niveau du congés 417a et du chanfrein 407, et une autre boucle autour de la paroi latérale 415a de la protubérance 415 au niveau du congés 419a et du chanfrein 407. Les parois latérales 413a, 415a des protubérances 413, 415 constituent ainsi des parois de déviations 413a, 415a du dispositif réducteur de tension 400. Dans une variante, les fils conducteurs 39a, 39b, 39c peuvent être enroulés plusieurs fois autour de la paroi latérale de chaque protubérance. Dans une autre variante, le nombre de tour des fils conducteurs 39a, 39b, 39c autour des parois de déviation 413a, 415a des protubérances 413, 415 peut être différent d'une protubérance à l'autre. De manière avantageuse, les fils conducteurs 39a, 39b, 39c et/ou des sections d'un même fil conducteur se superposent à eux-mêmes en certains points lors de l'agencement autour du dispositif réducteur de tension 400. Cela permet d'augmenter les frottements entre les portions superposées des fils conducteurs 39a, 39b, 39c et ainsi d'améliorer leur maintien et leur tenue en place.

Les fils conducteurs 39a, 39b, 39c sont ensuite regroupés en un fil multi-brin 40 au niveau de l'extrémité 405 du dispositif réducteur de tension 400.

L'agencement des fils conducteurs 39a, 39b, 39c autour des parois de déviation 413a, 415a du dispositif réducteur de tension 400 permet aux fils conducteurs 39a, 39b, 39c de pouvoir être bloqués lorsqu'ils sont sollicités en tension, c'est-à-dire que lorsque les fils conducteurs 39a, 39b, 39c sont sollicités en tension par une force représentée par une flèche F, ils se bloquent sur le dispositif réducteur de tension 200 contre les parois de déviation 413a, 415a des protubérances 413, 415. Cela permet notamment de réduire les sollicitations mécaniques subies par les fils conducteurs 39a, 39b, 39c en tension et donc de diminuer le risque de rupture des fils conducteurs 39a, 39b, 39c eux-mêmes ainsi qu'au niveau de la connexion électrique avec les hypotubes 41a, 41b, 41c du connecteur 19 de sonde 21.

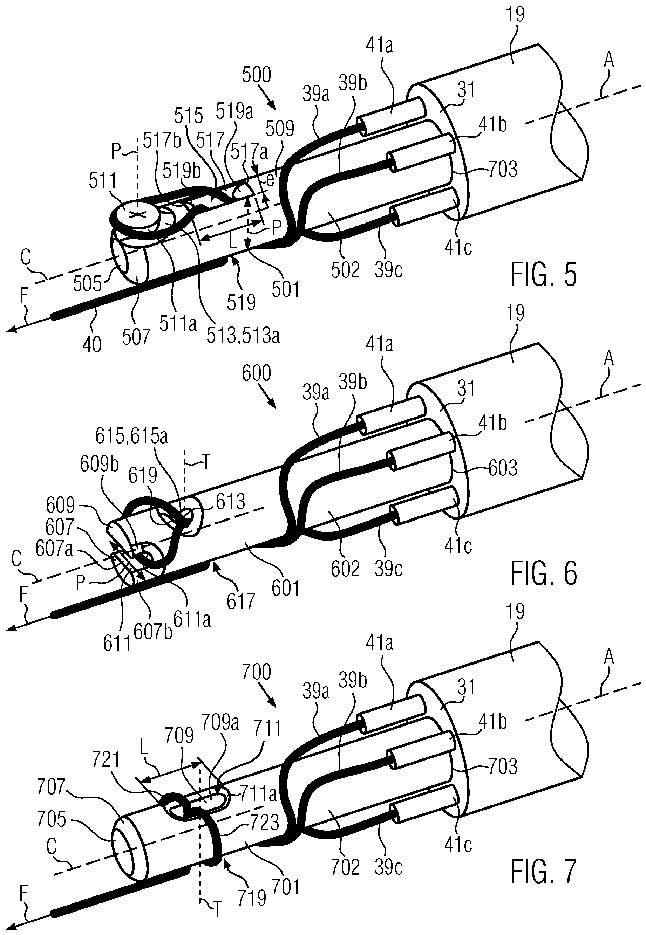

La **Figure 5** illustre une portion du connecteur 19 et un dispositif réducteur de tension 500 selon un cinquième mode de réalisation de la présente invention. Les éléments, en particulier relatifs au connecteur de sonde, avec les mêmes références numériques déjà utilisées pour la description des Figures 1 à 4 ne seront pas décrits à nouveau en détail, et référence est faite à leurs descriptions ci-dessus.

Le dispositif réducteur de tension 500 de la Figure 5 selon le cinquième mode de réalisation a une forme de cylindre circulaire droit 501 d'axe de révolution C. Une première extrémité 503 du cylindre 501 est attachée à l'extrémité proximale 31 du connecteur 19. Une seconde extrémité 505 du cylindre 501, opposée à l'extrémité 503, est représentée comme une extrémité libre 505 sur la Figure 5. L'axe de révolution C du dispositif réducteur de tension 500 est aligné avec l'axe A du connecteur 19. La section transversale de cylindre 501 est plus petite en tout point que la section transversale en tout point du connecteur 19.

L'extrémité libre 505 du dispositif réducteur de tension 500 est chanfreinée de manière à former une surface oblique 507 entre l'extrémité 405 et une paroi latérale 509 du cylindre 501. Cette surface chanfreinée 507 permet d'éviter une arête vive entre l'extrémité 505 et la paroi latérale 509 du cylindre 501. Ainsi, le chanfrein 507 permet de réduire le caractère potentiellement tranchant de l'arête entre l'extrémité 505 et la paroi latérale 509 du cylindre 501 qui pourrait endommager ou rompre les fils conducteurs 39a, 39b, 39c.

Selon le cinquième mode de réalisation, la paroi latérale 509 du cylindre 501 au niveau de l'extrémité 505 du dispositif réducteur de tension 400 est pourvue d'un premier élément de déviation, ici une protubérance cylindrique 511 de paroi latérale de déviation 551a et d'axe de révolution P. La protubérance 511 est positionnée de manière adjacente à l'extrémité 505 et tel que son axe de révolution P intersecte perpendiculairement l'axe C du cylindre 501. Ainsi, l'axe de révolution P du premier élément de déviation 511 est désaligné avec l'axe C du cylindre 501 du dispositif réducteur de tension 500. La paroi latérale de déviation 511a de la protubérance 511 a une jonction 513 avec la paroi latérale 509 du cylindre 501. La jonction 513 est pourvue d'un congés 513a afin d'arrondir l'arête entre la paroi latérale 509 et la protubérance 511. Le congés 513a permet ainsi de réduire la concentration de contrainte que créerait un angle vif au niveau de la jonction 513. Dans une variante, la paroi latérale 509 du cylindre 501 au niveau de l'extrémité 505 du dispositif réducteur de tension 400 pourrait aussi être pourvue de part et d'autre de la protubérance 511 des protubérances 413, 415 selon le quatrième mode de réalisation. Ainsi, le chanfrein 507 et le congés 513 du dispositif réducteur de tension 500 sont autant de caractéristiques structurelles qui permettent de réduire le risque d'endommager les fils conducteurs 39a, 39b, 39c.

Le dispositif réducteur de tension 500 comprend en outre un deuxième élément de déviation, ici un trou traversant 515 à proximité de la protubérance 511, plus précisément entre la protubérance 511 et l'extrémité 503. Le trou traversant 515, de longueur L et de largueur e, a une profondeur p telle que le trou traversant 515 traverse le cylindre 501 de part en part, et en particulier en intersectant l'axe C du cylindre 501. De ce fait, l'axe du trou traversant 515 qui s'étend le long de la profondeur p est désaligné avec l'axe C du cylindre 501 Le trou traversant 515 comprend deux entrées 517, 519 débouchant de part et d'autre du cylindre 501 telles que les entrées 517, 519 sont diamétralement opposées l'une de l'autre. Le trou traversant 515 est positionné telle que l'entrée 517 du trou 515 est alignée avec la protubérance 511.

Au niveau des entrées 517, 519, chaque arête 517a, 517b (seule l'arête 517a est visible sur la Figure 5) située entre la largeur e du trou traversant 515 et la paroi latérale 509 est pourvue d'un arrondi 519a, 519b c'est-à-dire d'une surface de raccordement arrondie 519a, 519b réalisant la jonction entre la largeur I du trou traversant 515 et la paroi latérale 509 de manière à éviter un angle vif qui pourrait endommager les fils conducteurs 39a, 39b, 39c. Dans une variante, les longueurs L de chaque entrée 517, 519 du trou traversant 515 peuvent aussi être chanfreinées ou arrondies.

Selon le cinquième mode de réalisation, les fils conducteurs 39a, 39b, 39c sont insérés par l'entrée 519 dans le trou traversant 515 et ressortent par l'entrée 517 de manière groupée en un fil multi-brin 40. Le fil multi-brin 40 est agencé en boucle autour de la paroi de déviation 511a de la protubérance 511 puis inséré dans le trou traversant 515 par l'entrée 517. Le fil multi-brin 40 ressort alors par l'entrée opposée 519 et est aligné avec l'axe C du cylindre 501 dans une direction orientée vers l'extrémité 505 du cylindre 501. Ainsi, les fils conducteurs 39a, 39b, 39c sont regroupés en un fil multi-brin 40 au niveau de l'extrémité 505 du dispositif réducteur de tension 500.

De ce fait, les fils conducteurs 39a, 39b, 39c sont déviés par le premier élément de déviation, c'est-à-dire par la protubérance 511 et le deuxième élément de déviation, c'est-à-dire le trou traversant 515 du dispositif réducteur de tension 300, de manière à ce que les éléments de déviations 511, 515 permettent de bloquer les fils conducteurs 39a, 39b, 39c contre la paroi de déviation 511a lorsqu'ils sont sollicités en tension par une force F. Les axes respectifs P, p des éléments de déviation 511, 515 s'étendent perpendiculairement par rapport à l'axe longitudinal C du dispositif réducteur de tension 500.

La **Figure 6** illustre une portion du connecteur 19 et un dispositif réducteur de tension 600 selon un sixième mode de réalisation de la présente invention. Les éléments, en particulier relatifs au connecteur de sonde, avec les mêmes références numériques déjà utilisées pour la description des Figures 1 à 5 ne seront pas décrits à nouveau en détail, et référence est faite à leurs descriptions ci-dessus.

Le dispositif réducteur de tension 600 de la Figure 6 selon le sixième mode de réalisation a la forme d'un cylindre 601 d'axe de révolution C et de paroi latérale 602. Une première extrémité 603 du cylindre 601 est attachée à l'extrémité proximale 31 du connecteur 19. Une seconde extrémité 605 du cylindre 601, opposée à l'extrémité 603, est une extrémité libre 605. L'axe de révolution C du dispositif réducteur de tension 600 est aligné avec l'axe A du connecteur 19. La section transversale de cylindre 601 est plus petite en tout point que la section transversale en tout point du connecteur 19.

L'extrémité libre 605 du cylindre 601 est pourvue de deux protubérances 607, 609 qui forment entre elles une rainure 611 qui a un fond 611a. Le fond 611a correspond à la jonction entre une paroi 607a de la protubérance 607 et une paroi (non visible sur la Figure 6) de la protubérance 609 faisant face à la paroi 607a. Les angles 607b, 609b des parois 607a, 609a sont chanfreinés ou arrondies de manière à former une surface oblique qui permet d'adoucir la jonction avec la rainure 611. Selon le sixième mode de réalisation, le fond 611a de la rainure 611 correspond à une paroi de déviation 611a du dispositif réducteur de tension 600. Le fond 611a de la rainure 611 s'étend longitudinalement selon une direction P qui est perpendiculaire à l'axe C du dispositif réducteur de tension 600.

Selon le sixième mode de réalisation, le dispositif réducteur de tension 600 comprend vers l'extrémité libre 605 un élément de déviation, ici un trou traversant 613 d'axe T pénétrant à travers le cylindre 601 tel qu'il y ait une intersection entre l'axe T du trou traversant 613 et l'axe C du cylindre 601. Ainsi, l'axe T du trou traversant 613 est désaligné avec l'axe C du cylindre 601. Le trou traversant 613 est dimensionné de manière à ce que les fils conducteurs 39a, 39b, 39c puissent être insérés de part et d'autre dans le trou 613 par une entrée 615 et une entrée 617. Chaque entrée 615, 617 du trou 613 a une jonction 615a (617a n'est pas visible sur la Figure 6) avec la paroi latérale 602 du cylindre 601. Les jonctions 615a, 617a du trou 613 sont chanfreinées ou arrondies de manière à adoucir la jonction avec la paroi latérale 602 du cylindre 601 et d'éviter d'endommager ou de rompre les fils conducteurs 39a, 39b, 39c. De plus, selon le sixième mode de réalisation, les jonctions 615a, 617a du trou 613 sont davantage chanfreinées de part et d'autre d'un diamètre D du trou 613 de manière à former un dénivelé 619 de part et d'autres des jonctions 615a, 617a. Ce dénivelé 619 permet d'améliorer le maintien des fils conducteurs 39a, 39b, 39c et de réduire les contraintes subies par les fils conducteurs 39a, 39b, 39c. Dans une variante, le trou traversant 613 pourrait aussi être utilisé à la place du trou traversant 515 décrit dans le cinquième mode de réalisation décrit ci-dessus par rapport à la Figure 5.

Dans autre une variante, la paroi latérale 602 du cylindre 601 peut être pourvue entre la rainure 611 et le trou traversant 613, d'une protubérance autour de laquelle les fils conducteurs 39a, 39b, 39c peuvent être enroulés, par exemple pourvue de la protubérance 511 du cinquième mode de réalisation.

Selon le sixième mode de réalisation, les fils conducteurs 39a, 39b, 39c sont insérés par l'entrée 617 dans le trou traversant 613 et ressort du trou traversant 613 par l'entrée 615. Les fils conducteurs 39a, 39b, 39c sont ensuite positionnés le long de la direction P sur la paroi de déviation 611a du fond de la rainure 611. Ainsi, les fils conducteurs 39a, 39b, 39c sont déviés de manière perpendiculaire par rapport à l'axe C du cylindre 601 du dispositif 600, étant donné que l'axe P de la paroi de déviation 611a est perpendiculaire à l'axe C. Les fils conducteurs 39a, 39b, 39c sont alors insérés par l'entrée 615 dans le trou traversant 613 et ressortent du trou traversant 613 par l'entrée 617 en un fil multi-brin 40. Ainsi, dans le cas où le fil multi-brin 40 est tiré par une force de tension F, lors de l'implantation de la sonde par exemple, les fils conducteurs 39a, 39b, 39c sont bloqués contre le fond 611a de la rainure 611 délimitée par les parois 607a, 609a des protubérances 607, 609, ce qui permet de les protéger de la sollicitation en tension. Cela permet aussi d'éviter que les fils conducteurs 39a, 39b, 39c ne soient tirés au niveau du raccordement avec les hypotubes 41a, 41b, 41c, ce qui pourrait casser la soudure entre les fils conducteurs et les hypotubes et donc endommager la connexion électrique.

De ce fait, les fils conducteurs 39a, 39b, 39c sont déviés par la rainure 611a et le trou traversant 613 du dispositif réducteur de tension 600 de manière à ce que les éléments de déviation 611, 613 permettent de bloquer les fils conducteurs 39a, 39b, 39c contre la paroi de déviation 611a lorsqu'ils sont sollicités en tension par une force F.

La **Figure 7** illustre une portion du connecteur 19 et un dispositif réducteur de tension 700 selon un septième mode de réalisation de la présente invention. Les éléments, en particulier relatifs au connecteur de sonde, avec les mêmes références numériques déjà utilisées pour la description des Figures 1 à 6 ne seront pas décrits à nouveau en détail, et référence est faite à leurs descriptions ci-dessus.

Le dispositif réducteur de tension 700 de la Figure 7 selon le septième mode de réalisation a une forme de cylindre circulaire droit 701 d'axe de révolution C et de paroi latérale 702. Une première extrémité 703 du cylindre 701 est attachée à l'extrémité proximale 31 du connecteur 19. Une seconde extrémité 705 du cylindre 701, opposée à l'extrémité 703, est représentée comme une extrémité libre 705 sur la Figure 7. L'axe de révolution C du dispositif réducteur de tension 700 est aligné avec l'axe A du connecteur 19. La section transversale de cylindre 701 est plus petite en tout point que la section transversale en tout point du connecteur 19.

L'extrémité libre 705 du dispositif réducteur de tension 700 est chanfreinée ou arrondie de manière à former une surface oblique 707 entre l'extrémité 705 et la paroi latérale 702 du cylindre 701. Cette surface chanfreinée 707 permet d'éviter une arête vive en adoucissant l'arête entre l'extrémité 705 et la paroi latérale 709 du cylindre 701. Ainsi, le chanfrein 707 permet d'éviter d'endommager ou rompre les fils conducteurs 39a, 39b, 39c.

Le cylindre 701 du dispositif réducteur de tension 700 comprend un élément de déviation, ici un trou oblong 709 traversant le cylindre 701 de part et d'autre de manière à ce que l'axe T qui s'étend le long de la profondeur du trou traversant oblong 709 intersecte l'axe C du cylindre 701. De ce fait, le trou traversant 709 comprend une paroi interne de déviation 709a qui s'étend selon l'axe T du trou oblong 709, c'est-à-dire de manière perpendiculaire à l'axe C du cylindre 701 du dispositif 700. Le trou traversant 709 est pourvu de deux entrées (ou sorties) 711, 719 au niveau de la paroi latérale 702, qui sont diamétralement opposées l'une de l'autre. Le trou traversant 709 est positionné de manière à ce que la plus grande longueur L du trou oblong traversant 709 soit parallèle à l'axe C du cylindre 701.

Chaque entrée 711, 719 du trou traversant 709 a une jonction 711a (719a n'est pas visible sur la Figure 7) avec la paroi latérale 702 du cylindre 701. Les jonctions 711a du trou traversant 709 sont chanfreinées ou arrondies de manière à adoucir la jonction avec la paroi latérale 702 du cylindre 701 et d'éviter d'endommager ou de rompre les fils conducteurs 39a, 39b, 39c.

Selon le septième mode de réalisation, les fils conducteurs 39a, 39b, 39c sont insérés par l'entrée 719 dans le trou oblong traversant 709 et ressortent du trou oblong traversant 709 par l'entrée 711. Ils sont ensuite enroulés dans un sens opposé sur un demi-tour de cylindre 701 afin d'être introduits à nouveau dans le trou oblong traversant 709 par l'entrée 719. Les fils conducteurs 39a, 39b, 39c sont ensuite sortis du trou oblong traversant 709 par l'entrée 719 et dirigés vers l'extrémité 705 du dispositif réducteur de tension 700.

Ainsi, les fils conducteurs 39a, 39b, 39c sont arrangés en deux boucles 721, 723 autour du trou oblong 709 du cylindre 701. De ce fait, lorsque les fils conducteurs 39a, 39b, 39c sont tirés par une force de tension F, lors de l'implantation de la sonde par exemple, les boucles 721, 723 de fils conducteurs se bloquent notamment contre une paroi interne 709a du trou oblong 709 dite paroi de déviation 709a, ce qui permet de les protéger de la sollicitation en tension. Cela permet ainsi d'éviter que les fils conducteurs 39a, 39b, 39c ne soient tirés au niveau du raccordement avec les hypotubes 41a, 41b, 41c, ce qui pourrait casser la soudure entre les fils conducteurs et les hypotubes et donc endommager la connexion électrique.

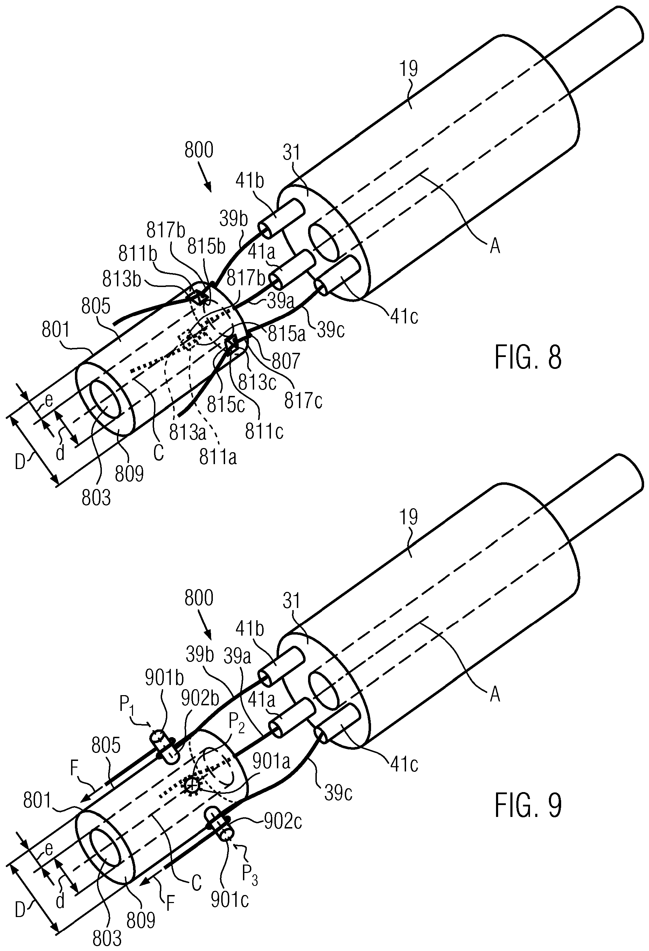

La **Figure 8** illustre une portion du connecteur 19 et un dispositif réducteur de tension 800 selon un huitième mode de réalisation de la présente invention. Les éléments, en particulier relatifs au connecteur de sonde, avec les mêmes références numériques déjà utilisées pour la description des Figures 1 à 7 ne seront pas décrits à nouveau en détail, et référence est faite à leurs descriptions ci-dessus.

Le dispositif réducteur de tension 800 est configuré pour s'adapter à différents types de sonde, telles que des sondes multi-lumen, des sondes avec une structure coaxiale ou coradiale etc. Selon le huitième mode de réalisation, le dispositif réducteur de tension 800 est attaché à l'extrémité proximale 31 du connecteur 19. Cependant, afin d'illustrer plus facilement la structure du dispositif réducteur de tension 800, la Figure 8 représente le dispositif réducteur de tension 800 de manière détachée du connecteur 19.

Le dispositif réducteur de tension 800 a la forme d'un tube 801 comprenant une partie creuse 803 d'axe C. Le dispositif réducteur de tension 800 est positionné de manière à ce que l'axe C soit aligné avec l'axe A du connecteur 19. Le tube 801 comprend une paroi latérale 805 qui s'étend selon l'axe C entre une extrémité 807 et une extrémité 809. L'extrémité 807 du tube 801 fait face à l'extrémité 31 du connecteur 19. La partie creuse 803, de section circulaire, a un diamètre d, aussi dit diamètre interne du tube 801. Le tube 801 a un diamètre externe D, tel qu'il y ait une épaisseur e entre le diamètre externe D et le diamètre interne d.

Au niveau de l'extrémité 807, le tube 801 est pourvu d'éléments de déviation qui sont au même nombre que les fils conducteurs 39a, 39b, 39c. Ainsi, selon le huitième mode de réalisation illustré par la Figure 8, le tube 801 est pourvu de trois trous traversant 811a, 811b, 811c chacun dimensionné pour un recevoir un fil conducteur 39a, 39b, 39c. Les trous traversant 811a, 811b, 811c traversent la paroi latérale 805 jusqu'à la partie creuse 803. De ce fait, les trous traversant 811a, 811b, 811c ont une profondeur équivalente à l'épaisseur e. Chacun des trous 811a, 811b, 811c comprend une première entrée 813a, 813b, 813c affleurant la paroi latérale 805 et une deuxième entrée 815a, 815b, 815c débouchant sur la partie creuse 803.

Selon le septième mode de réalisation, les fils conducteurs 39a, 39b, 39c sont introduits dans la partie creuse 803 par l'extrémité 807 puis chaque fil conducteur 39a, 39b, 39c est inséré dans le trou traversant correspondant 811a, 811b, 811c par l'entrée 815a, 815b, 815c et ressort par l'entrée 813a, 813b, 813c. Chaque fil conducteur 39a, 39b, 39c est de nouveau inséré dans la partie creuse 803 par l'extrémité 807 de manière à former une boucle 817a, 817b, 817c. Chaque fil conducteur 39a, 39b, 39c est ensuite inséré dans le trou traversant correspondant 811a, 811b, 811c par l'entrée 815a, 815b, 815c et ressort par l'entrée 813a, 813b, 813c. De cette manière, chaque fils conducteur 39a, 39b, 39c est individuellement protégé d'une sollicitation en tension car lorsqu'un fil conducteur 39a, 39b, 39c est tiré cela provoque un blocage de la boucle 817a, 817b, 817c autour de l'extrémité 807 du tube 801. Cela permet ainsi d'éviter que les fils conducteurs 39a, 39b, 39c ne soient sollicités en tension au niveau du raccordement avec les hypotubes 41a, 41b, 41c, ce qui pourrait casser la soudure entre les fils conducteurs et les hypotubes et donc endommager la connexion électrique.

La **Figure 9** illustre une portion du connecteur 19 et un dispositif réducteur de tension 900 selon un neuvième mode de réalisation de la présente invention. Les éléments avec les mêmes références numériques déjà utilisées pour la description des Figures 1 à 8 ne seront pas décrits à nouveau en détail, et référence est faite à leurs descriptions ci-dessus.

De manière similaire au huitième mode de réalisation, le dispositif réducteur de tension 900 du neuvième mode de réalisation est configuré pour s'adapter à différent type de sondes, telles que des sondes multi lumen, des sondes avec une structure coaxiale ou coradiale etc. Ainsi seuls les éléments structurels qui différent de ceux déjà décrits pour le dispositif réducteur de tension 800 seront décrits.

A la différence du dispositif réducteur de tension 800 décrit à la Figure 8, le tube 801 du dispositif réducteur de tension 900 ne comprend pas de trous traversant comme éléments de déviation mais des protubérances cylindriques 901a, 901b, 901c au niveau de l'extrémité 807 du tube 801.

Ces protubérances cylindriques 901a, 901b, 901c font saillie depuis la paroi latérale 805 du cylindre 801 de manière à ce que leurs axes de révolution respectifs P1, P2, P3 intersectent l'axe C du cylindre 801 en en même point. Ainsi, les parois latérales 902a, 902b, 902c des protubérances 901a, 901b, 901c s'étendent de manière perpendiculaire la paroi latérale 805 du cylindre 801. Dans le neuvième mode de réalisation, les parois latérales 902a, 902b, 902c des protubérances 901a, 901b, 901c sont les parois de déviation 902a, 902b, 902c du dispositif réducteur de tension 900.

Selon le neuvième mode de réalisation, les fils conducteurs 39a, 39b, 39c sont chacun agencés autour des parois latérales 902a, 902b, 902c des protubérances 901a, 901b, 901c du tube 801. Ainsi, dans le cas où les fils conducteurs 39a, 39b, 39c sont tirés par une force de tension F, lors de l'implantation de la sonde par exemple, chaque fil conducteur 39a, 39b, 39c se bloquent autour de la protubérance 901a, 901b, 901c respective ce qui permet de les protéger de la sollicitation en tension. Cela permet ainsi d'éviter que les fils conducteurs 39a, 39b, 39c ne soient tirés au niveau du raccordement avec les hypotubes 41a, 41b, 41c, ce qui pourrait casser la soudure entre les fils conducteurs et les hypotubes et donc endommager la connexion électrique.

Les modes de réalisation décrits sont simplement des configurations possibles et il faut garder à l'esprit que les caractéristiques individuelles des neufs modes de réalisation peuvent être combinées entre elles ou fournies indépendamment les unes des autres. De plus les géométries montrées dans les figures 2 à 9 peuvent être variées sans partir des concepts des inventions. Dans une variante, représentée à la Figure 3 et applicable à tous les modes de réalisation, chacun des fils conducteurs peut d'abord être soudé à un hypotube intermédiaire de diamètre plus petit que le hypotube du connecteur avant d'être inséré dans l'hypotube du conducteur. En particulier, pour des diamètres de fil conducteur plus petits que 150 micromètres, l'utilisation d'hypotubes intermédiaires, tel que décrit dans le mode de réalisation de la Figure 3, peut améliorer la longévité de la sonde.

## Revendications

1. Sonde implantable comprenant au moins un fil conducteur (39a, 39b, 39c, 40) et un connecteur (19), configuré pour être connecté à un dispositif médical implantable (100) de type dispositif de stimulation cardiaque, de défibrillation ou/et de neuromodulation, et comprenant en outre un dispositif réducteur de tension (200, 300, 400, 500, 600, 700, 800, 900) qui s'étend longitudinalement selon un axe (C) à partir d'une extrémité (31) du connecteur (19) et essentiellement parallèle à l'axe longitudinal (A) du connecteur (19) et qui comprend au moins un élément (211, 219, 307, 313, 413, 415, 511, 515, 611, 613, 709, 811a-c, 901a-c) avec une paroi de déviation (215, 313a, 413a, 415a, 511a, 611a, 709a);
tel que le fil conducteur (39a, 39b, 39c, 40) est dévié par la paroi de déviation (215, 313a, 413a, 415a, 511a, 611a, 709a) du dispositif réducteur de tension (200, 300, 400, 500, 600, 700, 800, 900) de manière à ce que l'élément de déviation (211, 219, 307, 313, 413, 415, 511, 515, 611, 613, 709, 811a-c, 901a-c) du dispositif réducteur de tension (200, 300, 400, 500, 600, 700, 800, 900) bloque le fil conducteur (39a, 39b, 39c, 40) contre la paroi de déviation (215, 313a, 413a, 415a, 511a, 611a, 709a) lorsque le fil conducteur (39a, 39b, 39c, 40) est sollicité en tension ; et
la paroi de déviation (215, 313a, 413a, 415a, 511a, 611a, 709a) s'étend selon un axe (P, P', T) qui est désaligné avec l'axe longitudinal (C) du dispositif réducteur de tension (200, 300, 400, 500, 600, 700, 800, 900).

2. La sonde implantable selon la revendication 1, dont l'élément de déviation (219, 307, 313, 413, 415, 511, 515, 611, 613, 709, 811a-c) comprend un trou traversant (307, 515, 613, 709, 811a-c) dont la profondeur s'étend selon un axe (T) désaligné avec l'axe longitudinal (C) du dispositif réducteur de tension (200, 300, 400, 500, 600, 700, 800).

3. La sonde implantable selon la revendication 1 ou 2, dont l'élément de déviation (219, 611) comprend une encoche (219, 611) dont la profondeur s'étend selon un axe (T) désaligné avec l'axe longitudinal (C) du dispositif réducteur de tension (200, 600).

4. La sonde implantable selon l'une des revendications 1 à 3, dont l'élément de déviation (413, 415, 511, 901a-c) comprend une protubérance (413, 415, 511, 901a-c) qui fait saillie depuis le dispositif réducteur de tension selon un axe (P, P') désaligné avec l'axe longitudinal (C) du dispositif réducteur de tension (400, 500, 900).

5. La sonde implantable selon l'une des revendications 2 à 4, dont au moins une section du fil conducteur (39a, 39b, 39c, 40) est agencée en boucle autour de la paroi de déviation (413a, 415a, 511a, 901a-c) d'une protubérance (413, 415, 511, 901a-c) qui fait saillie depuis une surface extérieure du dispositif réducteur de tension (400, 500, 900).

6. La sonde implantable selon la revendication 5, dans laquelle la protubérance (413, 415, 511, 901a-c) a une forme cylindrique et un axe de révolution (P) désaligné avec l'axe longitudinal (C) du dispositif réducteur de tension (400, 500, 900).

7. La sonde implantable selon l'une des revendications 2 à 4, dont le fil conducteur (39a, 39b, 39c, 40) est introduit au moins deux fois à travers le trou traversant (307, 515, 613, 709, 811) de manière à ce que le fil conducteur (39a, 39b, 39c, 40) est agencé en boucle (721, 723) autour d'une portion (313) du dispositif réducteur de tension (300, 500, 600, 700, 800).

8. La sonde implantable selon l'une des revendications précédentes, dont la paroi de déviation (215, 313a, 413a, 415a, 511a, 611a, 709a) s'étend selon un axe (P, P', T) qui est désaligné avec l'axe longitudinal (C) du dispositif réducteur de tension (200, 300, 400, 500, 600, 700, 800, 900) de manière à former un angle droit à plus ou moins 10 degrés.

9. La sonde implantable selon l'une de revendications précédentes, dont au moins deux sections du fil conducteur (39a, 39b, 39c, 40) se chevauchent ou se superposent.

10. La sonde implantable selon l'une des revendications précédentes, dont le connecteur (19) et le dispositif de réducteur de tension (200, 300, 400, 500, 600, 700, 800, 900) sont chacun pourvus d'un ou de plusieurs lumens ; au moins un lumen du dispositif de réducteur de tension (200, 300, 400, 500, 600, 700, 800, 900) étant aligné avec un lumen du connecteur (19).

11. La sonde implantable selon l'une des revendications précédentes, comprenant plusieurs fils conducteurs (39a, 39b, 39c) tel que chaque fil conducteur (39a, 39b, 39c) est dévié par le dispositif réducteur de tension (200, 300, 400, 500, 600, 700, 800, 900) de manière à ce que l'élément de déviation (211, 219, 307, 313, 413, 415, 511, 515, 607, 613, 709, 811a-c, 901a-c) du dispositif réducteur de tension (200, 300, 400, 500, 600, 700, 800, 900) bloque les fil conducteur (39a, 39b, 39c, 40) lorsque les fils conducteurs (39a, 39b, 39c, 40) sont sollicités en tension.

12. La sonde implantable selon la revendication 11, dont les fils conducteurs (39a, 39b, 39c) sortent d'une extrémité (31) du connecteur (19) de manière distincte les uns des autres et sont regroupés en un fil multi-brin (40) par leur agencement autour du dispositif réducteur de tension (200, 300, 400, 500, 600, 700).

13. La sonde implantable selon l'une des revendications précédentes, dont le dispositif réducteur de tension (200, 300, 400, 500, 600, 700) est attaché à une extrémité (31) du connecteur (19) par collage, par emboîtement, par raccordement encliquetable ou par un ou des éléments de fixation.

14. La sonde implantable selon l'une des revendications 1 à 13, dont le dispositif réducteur de tension (200, 300, 400, 500, 600, 700) et le au moins un élément de déviation (211, 219, 307, 313, 413, 415, 511, 515, 607, 613, 709, 811a-c, 901a-c) sont formés de manière intégrale avec le connecteur (19).

15. Un dispositif médical implantable de type dispositif de stimulation cardiaque, de défibrillation ou/et de neuromodulation comprenant une sonde implantable (21) selon la revendication 1, elle-même comprenant le connecteur (19), le dispositif réducteur de tension (200, 300, 400, 500, 600, 700, 800, 900) d'axe longitudinal (C) et le au moins fil conducteur (39a, 39b, 39c, 40) connecté électriquement au connecteur (19);
tel que le dispositif réducteur de tension s'étend longitudinalement à partir d'une extrémité (31) du connecteur (19) selon un axe (C) et qui est essentiellement parallèle à l'axe longitudinal (A) du connecteur (19), et comprend au moins un élément (211, 219, 307, 313, 413, 415, 511, 515, 607, 613, 709, 811a-c, 901a-c) avec une paroi de déviation (215, 313a, 413a, 415a, 511a, 611a, 709a);
le connecteur (19) et le dispositif réducteur de tension (200, 300, 400, 500, 600, 700, 800, 900) étant configurés pour être logés dans le dispositif médical implantable (100) ;
de sorte que le fil conducteur (39a, 39b, 39c, 40) est dévié par la paroi de déviation (215, 313a, 413a, 415a, 511a, 611a, 709a) du dispositif réducteur de tension (200, 300, 400, 500, 600, 700, 800, 900) de manière à ce que la paroi de déviation (215, 313a, 413a, 415a, 511a, 611a, 709a) du dispositif réducteur de tension (200, 300, 400, 500, 600, 700, 800, 900) bloque le fil conducteur (39a, 39b, 39c, 40) lorsque le fil conducteur (39a, 39b, 39c, 40) est sollicité en tension ; et
la paroi de déviation (215, 313a, 413a, 415a, 511a, 611a, 709a) s'étend selon un axe (P, P', T) qui est désaligné avec l'axe longitudinal (C) du dispositif réducteur de tension (200, 300, 400, 500, 600, 700, 800, 900).

16. Méthode de fabrication d'une sonde implantable configurée pour être connectée au moyen d'un fil ou de plusieurs fils conducteurs (39a, 39b, 39c, 40) et d'un connecteur (19) à un dispositif médical implantable (100) de type dispositif de stimulation cardiaque, de défibrillation ou/et de neuromodulation,
la sonde implantable (21) comprenant en outre un dispositif réducteur de tension (200, 300, 400, 500, 600, 700, 800, 900) d'axe longitudinal (C) pourvu à une extrémité (31) du connecteur (19),
le dispositif réducteur de tension (200, 300, 400, 500, 600, 700, 800, 900) comprenant lui-même au moins un élément avec une paroi de déviation (215, 313a, 413a, 415a, 511a, 611a, 709a);
ladite méthode comprenant une étape de déviation du ou des fils conducteurs (39a, 39b, 39c, 40) par le dispositif réducteur de tension de manière à agencer le ou les fils conducteurs (39a, 39b, 39c, 40) du connecteur (19) autour de la paroi de déviation (215, 313a, 413a, 415a, 511a, 611a, 709a) qui s'étend selon un axe (P, P', T) qui est désaligné avec l'axe longitudinal (C) du dispositif réducteur de tension (200, 300, 400, 500, 600, 700, 800, 900) ;
de manière à ce que lorsque le ou les fils conducteurs (39a, 39b, 39c, 40) sont sollicités en tension, le ou les fils conducteurs (39a, 39b, 39c, 40) se bloquent contre la paroi de déviation (215, 313a, 413a, 415a, 511a, 611a, 709a) du dispositif réducteur de tension (200, 300, 400, 500, 600, 700, 800, 900).

## Patentansprüche

1. Implantierbare Sonde, umfassend mindestens einen Leitungsdraht (39a, 39b, 39c, 40) und einen Steckverbinder (19), der für den Anschluss an eine implantierbare medizinische Vorrichtung (100) der Art einer Herzstimulationsvorrichtung, einer Defibrillationsvorrichtung und/oder einer Neuromodulationsvorrichtung konfiguriert ist, und umfassend unter anderem eine Zugentlastungsvorrichtung (200, 300, 400, 500, 600, 700, 800, 900), die sich in Längsrichtung entlang einer Achse (C) ab einem Ende (31) des Steckverbinders (19) und im Wesentlichen parallel zur Längsachse (A) des Steckverbinders (19) erstreckt und die mindestens ein Element (211, 219, 307, 313, 413, 415, 511, 515, 611, 613, 709, 811a-c, 901a-c) mit einer Umlenkwand (215, 313a, 413a, 415a, 511a, 611a, 709a) umfasst;
wobei der Leitungsdraht (39a, 39b, 39c, 40) über die Umlenkwand (215, 313a, 413a, 415a, 511a, 611a, 709a) der Zugentlastungsvorrichtung (200, 300, 400, 500, 600, 700, 800, 900) derart umgelenkt wird, dass das Umlenkelement (211, 219, 307, 313, 413, 415, 511, 515, 611, 613, 709, 811a-c, 901a-c) der Zugentlastungsvorrichtung (200, 300, 400, 500, 600, 700, 800, 900) den Leitungsdraht (39a, 39b, 39c, 40) an der Umlenkwand (215, 313a, 413a, 415a, 511a, 611a, 709a) arretiert, wenn der Leitungsdraht (39a, 39b, 39c, 40) zugbeansprucht wird; und
die Umlenkwand (215, 313a, 413a, 415a, 511a, 611a, 709a) sich entlang einer Achse (P, P', T) erstreckt, die zur Längsachse (C) der Zugentlastungsvorrichtung (200, 300, 400, 500, 600, 700, 800, 900) versetzt ist.

2. Die implantierbare Sonde nach Anspruch 1, wobei das Umlenkelement (219, 307, 313, 413, 415, 511, 515, 611, 613, 709, 811a-c) eine Duchgangsbohrung (307, 515, 613, 709, 811a-c) umfasst, deren Tiefe sich entlang einer Achse (T) erstreckt, die zur Längsachse (C) der Zugentlastungsvorrichtung (200, 300, 400, 500, 600, 700, 800) versetzt ist.

3. Die implantierbare Sonde nach Anspruch 1 oder 2, wobei das Umlenkelement (219, 611) eine Einkerbung (219, 611) umfasst, deren Tiefe sich entlang einer Achse (T) erstreckt, die zur Längsachse (C) der Zugentlastungsvorrichtung (200, 600) versetzt ist.

4. Die implantierbare Sonde nach einem der Ansprüche 1 bis 3, wobei das Umlenkelement (413, 415, 511, 901a-c) einen Vorsprung (413, 415, 511, 901a-c) umfasst, der über die Zugentlastungsvorrichtung entlang einer Achse (P, P'), die zur Längsachse (C) der Zugentlastungsvorrichtung (400, 500, 900) versetzt ist, hinausragt.

5. Die implantierbare Sonde nach einem der Ansprüche 2 bis 4, wobei mindestens ein Abschnitt des Leitungsdrahts (39a, 39b, 39c, 40) in einer Schleife um die Umlenkwand (413a, 415a, 511a, 901a-c) eines Vorsprungs (413, 415, 511, 901a-c), der über eine Außenfläche der Zugentlastungsvorrichtung (400, 500, 900) hinausragt, angeordnet ist.

6. Die implantierbare Sonde nach Anspruch 5, wobei der Vorstand (413, 415, 511, 901a-c) eine zylindrische Form und eine Rotationsachse (P), die zur Längsachse (C) der Zugentlastungsvorrichtung (400, 500, 900) versetzt ist, aufweist.

7. Die implantierbare Sonde nach einem der Ansprüche 2 bis 4, wobei der Leitungsdraht (39a, 39b, 39c, 40) mindestens zweimal derart durch die Durchgangsbohrung (307, 515, 613, 709, 811) eingeführt wird, dass der Leitungsdraht (39a, 39b, 39c, 40) in einer Schleife (721, 723) um einen Teil (313) der Zugentlastungsvorrichtung (300, 500, 600, 700, 800) angeordnet wird.

8. Die implantierbare Sonde nach einem der vorhergehenden Ansprüche, wobei die Umlenkwand (215, 313a, 413a, 415a, 511a, 611a, 709a) sich entlang einer Achse (P, P', T) erstreckt, die zur Längsachse (C) der Zugentlastungsvorrichtung (200, 300, 400, 500, 600, 700, 800, 900) derart versetzt ist, dass ein rechter Winkel plus oder minus 10 Grad gebildet wird.

9. Die implantierbare Sonde nach einem der vorhergehenden Ansprüche, wobei mindestens zwei Abschnitte des Leitungsdrahts (39a, 39b, 39c, 40) sich überschneiden oder übereinanderliegen.

10. Die implantierbare Sonde nach einem der vorhergehenden Ansprüche, wobei der Steckverbinder (19) und die Zugentlastungsvorrichtung (200, 300, 400, 500, 600, 700, 800, 900) jeweils mit einem oder mehreren Lumen ausgestattet sind; wobei mindestens ein Lumen der Zugentlastungsvorrichtung (200, 300, 400, 500, 600, 700, 800, 900) mit einem Lumen des Steckverbinders (19) ausgerichtet ist.

11. Die implantierbare Sonde nach einem der vorhergehenden Ansprüche, umfassend mehrere Leitungsdrähte (39a, 39b, 39c), wobei jeder Leitungsdraht (39a, 39b, 39c) von der Zugentlastungsvorrichtung (200, 300, 400, 500, 600, 700, 800, 900) derart umgelenkt wird, dass das Umlenkelement (211, 219, 307, 313, 413, 415, 511, 515, 607, 613, 709, 811a-c, 901a-c) der Zugentlastungsvorrichtung (200, 300, 400, 500, 600, 700, 800, 900) die Leitungsdrähte (39a, 39b, 39c, 40) arretiert, wenn die Leitungsdrähte (39a, 39b, 39c, 40) zugbeansprucht werden.

12. Die implantierbare Sonde nach Anspruch 11, wobei die Leitungsdrähte (39a, 39b, 39c) getrennt voneinander aus einem Ende (31) des Steckverbinders (19) austreten und durch ihre Anordnung um die Zugentlastungsvorrichtung (200, 300, 400, 500, 600, 700) in einem Mehrdrahtleiter (40) zusammengefasst werden.

13. Die implantierbare Sonde nach einem der vorhergehenden Ansprüche, wobei die Zugentlastungsvorrichtung (200, 300, 400, 500, 600, 700) an einem Ende (31) des Steckverbinders (19) durch Verkleben, Steckverbindung, Einrastverbindung oder durch ein oder mehrere Befestigungselemente befestigt wird.

14. Die implantierbare Sonde nach einem der Ansprüche 1 bis 13, wobei die Zugentlastungsvorrichtung (200, 300, 400, 500, 600, 700) und das mindestens eine Umlenkelement (211, 219, 307, 313, 413, 415, 511, 515, 607, 613, 709, 811a-c, 901a-c) einstückig mit dem Steckverbinder (19) ausgebildet sind.

15. Eine implantierbare medizinische Vorrichtung von der Art einer Herzstimulationsvorrichtung, einer Defibrillationsvorrichtung und/oder einer Neuromodulationsvorrichtung, umfassend eine implantierbare Sonde (21) nach Anspruch 1, die wiederum den Steckverbinder (19), die Zugentlastungsvorrichtung (200, 300, 400, 500, 600, 700, 800, 900) mit der Längsachse (C) und den mindestens einen Leitungsdraht (39a, 39b, 39c, 40), der elektrisch mit dem Steckverbinder (19) verbunden ist, umfasst; wobei die Zugentlastungsvorrichtung sich in Längsrichtung von einem Ende (31) des Steckverbinders (19) entlang einer Achse (C) erstreckt, die im Wesentlichen parallel zur Längsachse (A) des Steckverbinders (19) ist, und mindestens ein Element (211, 219, 307, 313, 413, 415, 511, 515, 607, 613, 709, 811a-c, 901a-c) mit einer Umlenkwand (215, 313a, 413a, 415a, 511a, 611a, 709a) umfasst; wobei der Steckverbinder (19) und die Zugentlastungsvorrichtung (200, 300, 400, 500, 600, 700, 800, 900) konfiguriert sind, um in der implantierbaren Vorrichtung (100) untergebracht zu werden;
sodass der Leitungsdraht (39a, 39b, 39c, 40) von der Umlenkwand (215, 313a, 413a, 415a, 511a, 611a, 709a) der Zugentlastungsvorrichtung (200, 300, 400, 500, 600, 700, 800, 900) derart umgelenkt wird, dass die Umlenkwand (215, 313a, 413a, 415a, 511a, 611a, 709a) der Zugentlastungsvorrichtung (200, 300, 400, 500, 600, 700, 800, 900) den Leitungsdraht (39a, 39b, 39c, 40) arretiert, wenn der Leitungsdraht (39a, 39b, 39c, 40) zugbeansprucht wird; und
die Umlenkwand (215, 313a, 413a, 415a, 511a, 611a, 709a) sich entlang einer Achse (P, P', T) erstreckt, die zur Längsachse (C) der Zugentlastungsvorrichtung (200, 300, 400, 500, 600, 700, 800, 900) versetzt ist.

16. Verfahren zur Herstellung einer implantierbaren Sonde, die konfiguriert ist, um mittels eines oder mehrerer Leitungsdrähte (39a, 39b, 39c, 40) und eines Steckverbinders (19) an eine implantierbare medizinische Vorrichtung (100) der Art einer Herzstimulationsvorrichtung, einer Defibrillationsvorrichtung und/oder einer Neuromodulationsvorrichtung angeschlossen zu werden,
wobei die implantierbare Sonde (21) unter anderem eine Zugentlastungsvorrichtung (200, 300, 400, 500, 600, 700, 800, 900) mit einer Längsachse (C), die an einem Ende (31) des Steckverbinders (19) vorgesehen ist, umfasst,
wobei die Zugentlastungsvorrichtung (200, 300, 400, 500, 600, 700, 800, 900) wiederum mindestens ein Element mit einer Umlenkwand (215, 313a, 413a, 415a, 511a, 611a, 709a) umfasst;
wobei besagtes Verfahren einen Schritt der Umlenkung des oder der Leitungsdrähte (39a, 39b, 39c, 40) durch die Zugentlastungsvorrichtung derart umfasst, dass der oder die Leitungsdrähte (39a, 39b, 39c, 40) des Steckverbinders (19) um die Umlenkwand (215, 313a, 413a, 415a, 511a, 611a, 709a) angeordnet werden, die sich entlang einer Achse (P, P', T) erstreckt, die zur Längsachse (C) der Zugentlastungsvorrichtung (200, 300, 400, 500, 600, 700, 800, 900) versetzt ist;
sodass, wenn der oder die Leitungsdrähte (39a, 39b, 39c, 40) zugbeansprucht werden, der oder die Leitungsdrähte (39a, 39b, 39c, 40) an der Umlenkwand (215, 313a, 413a, 415a, 511a, 61 1a, 709a) der Zugentlastungsvorrichtung (200, 300, 400, 500, 600, 700, 800, 900) arretiert werden.

## Claims

1. An implantable probe comprising at least one conductor wire (39a, 39b, 39c, 40) and a connector (19), configured to be connected to an implantable medical device (100) of the cardiac stimulation, defibrillation or/and neuromodulation device type, and further comprising a tension reducing device (200, 300, 400, 500, 600, 700, 800, 900) that extends longitudinally along an axis (C) from one end (31) of the connector (19) and essentially parallel to the longitudinal axis (A) of the connector (19) and that comprises at least one element (211, 219, 307, 313, 413, 415, 511, 515, 611, 613, 709, 811a-c, 901a-c) having a deflection wall (215, 313a, 413a, 415a, 511a, 611a, 709a);
wherein the conductor wire (39a, 39b, 39c, 40) is deflected by the deflection wall (215, 313a, 413a, 415a, 511a, 611a, 709a) of the tension reducing device (200, 300, 400, 500, 600, 700, 800, 900) such that the deflection element (211, 219, 307, 313, 413, 415, 511, 515, 611, 613, 709, 811a-c, 901a-c) of the tension reducing device (200, 300, 400, 500, 600, 700, 800, 900) blocks the conductor wire (39a, 39b, 39c, 40) against the deflection wall (215, 313a, 413a, 415a, 511a, 611a, 709a) when the conductor wire (39a, 39b, 39c, 40) is stressed in tension; and
the deflection wall (215, 313a, 413a, 415a, 511a, 611a, 709a) extends along an axis (P, P', T) that is out of alignment with the longitudinal axis (C) of the tension reducing device (200, 300, 400, 500, 600, 700, 800, 900).

2. The implantable probe of claim 1, wherein the deflection element (219, 307, 313, 413, 415, 511, 515, 611, 613, 709, 811a-c) comprises a through hole (307, 515, 613, 709, 811a-c) having its depth extending along an axis (T) that is out of alignment with the longitudinal axis (C) of the tension reducing device (200, 300, 400, 500, 600, 700, 800).

3. The implantable probe of claim 1 or claim 2, wherein the deflection element (219, 611) comprises a notch (219, 611) having its depth extending along an axis (T) that is out of alignment with the longitudinal axis (C) of the tension reducing device (200, 600).

4. The implantable probe of any one of claims 1 to 3, wherein the deflection element (413, 415, 511, 901a-c) comprises a protuberance (413, 415, 511, 901a-c) that projects from the tension reducing device along an axis (P, P') that is out of alignment with the longitudinal axis (C) of the tension reducing device (400, 500, 900).

5. The implantable probe of any one of claims 2 to 4, wherein at least one section of the conductor wire (39a, 39b, 39c, 40) is arranged in a loop around the deflection wall (413a, 415a, 511a, 901a-c) of a protuberance (413, 415, 511, 901a-c) that projects from an outer surface of the tension reducing device (400, 500, 900).

6. The implantable probe of claim 5, wherein the protuberance (413, 415, 511, 901a-c) has a cylindrical shape and an axis of revolution (P) that is out of alignment with the longitudinal axis (C) of the tension reducing device (400, 500, 900).

7. The implantable probe of any one of claims 2 to 4, wherein the conductor wire (39a, 39b, 39c, 40) is inserted at least twice through the through hole (307, 515, 613, 709, 811) such that the conductor wire (39a, 39b, 39c, 40) is arranged in a loop (721, 723) around a portion (313) of the tension reducing device (300, 500, 600, 700, 800).

8. The implantable probe of any one of the preceding claims, wherein the deflection wall (215, 313a, 413a, 415a, 511a, 611a, 709a) extends along an axis (P, P', T) that is out of alignment with the longitudinal axis (C) of the tension reducing device (200, 300, 400, 500, 600, 700, 800, 900) in such a manner as to form a right angle to within plus or minus 10 degrees.

9. The implantable probe of any one of the preceding claims, wherein at least two sections of the conductor wire (39a, 39b, 39c, 40) overlap one another or are superimposed on one another.

10. The implantable probe of any one of the preceding claims, wherein the connector (19) and the tension reducing device (200, 300, 400, 500, 600, 700, 800, 900) are each provided with one or more lumens; at least one lumen of the tension reducing device (200, 300, 400, 500, 600, 700, 800, 900) being aligned with a lumen of the connector (19).

11. The implantable probe of any one of the preceding claims, including a plurality of conductor wires (39a, 39b, 39c), each conductor wire (39a, 39b, 39c) being deflected by the tension reducing device (200, 300, 400, 500, 600, 700, 800, 900) such that the deflection element (211, 219, 307, 313, 413, 415, 511, 515, 607, 613, 709, 811a-c, 901a-c) of the tension reducing device (200, 300, 400, 500, 600, 700, 800, 900) blocks the conductor wires (39a, 39b, 39c, 40) when the conductor wires (39a, 39b, 39c, 40) are stressed in tension.

12. The implantable probe of claim 11, wherein the conductor wires (39a, 39b, 39c) exit from one end (31) of the connector (19) separately from one another and are grouped together into a multi-stranded wire (40) by being arranged around the tension reducing device (200, 300, 400, 500, 600, 700).

13. The implantable probe of any one of the preceding claims, wherein the tension reducing device (200, 300, 400, 500, 600, 700) is attached to one end (31) of the connector (19) by adhesive bonding, by interfitting engagement, by snap-fastening or by one or more fastening elements.

14. The implantable probe of any one of claims 1 to 13, wherein the tension reducing device (200, 300, 400, 500, 600, 700) and the at least one deflection element (211, 219, 307, 313, 413, 415, 511, 515, 607, 613, 709, 811a-c, 901a-c) are formed integrally with the connector (19).

15. An implantable medical device of the cardiac stimulation, defibrillation and/or neuromodulation type including an implantable probe (21) according to claim 1, itself comprising a connector (19), a tension reducing device (200, 300, 400, 500, 600, 700, 800, 900) of longitudinal axis (C) and at least one conductor wire (39a, 39b, 39c, 40) electrically connected to the connector (19); wherein the tension reducing device extends longitudinally from one end (31) of the connector (19) along an axis (C) and is essentially parallel to the longitudinal axis (A) of the connector (19), and comprises at least one element (211, 219, 307, 313, 413, 415, 511, 515, 607, 613, 709, 811a-c, 901a-c) having a deflection wall (215, 313a, 413a, 415a, 511a, 611a, 709a);
the connector (19) and the tension reducing device (200, 300, 400, 500, 600, 700, 800, 900) being configured to be housed in the implantable medical device (100);
and wherein the conductor wire (39a, 39b, 39c, 40) is deflected by the deflection wall (215, 313a, 413a, 415a, 511a, 611a, 709a) of the tension reducing device (200, 300, 400, 500, 600, 700, 800, 900) such that the deflection wall (215, 313a, 413a, 415a, 511a, 611a, 709a) of the tension reducing device (200, 300, 400, 500, 600, 700, 800, 900) blocks the conductor wire (39a, 39b, 39c, 40) when the conductor wire (39a, 39b, 39c, 40) is stressed in tension; and
the deflection wall (215, 313a, 413a, 415a, 511a, 611a, 709a) extends along an axis (P, P', T) that is out of alignment with the longitudinal axis (C) of the tension reducing device (200, 300, 400, 500, 600, 700, 800, 900).

16. A method of manufacturing an implantable probe configured to be connected by means of one or more conductor wires (39a, 39b, 39c, 40) and of a connector (19) to an implantable medical device (100) of the cardiac stimulation, defibrillation or/and neuromodulation device type.
the implantable probe (21) further comprising a tension reducing device (200, 300, 400, 500, 600, 700, 800, 900) of longitudinal axis (C) and provided at one end (31) of the connector (19),
the tension reducing device (200, 300, 400, 500, 600, 700, 800, 900) itself comprising at least one element having a deflection wall (215, 313a, 413a, 415a, 511a, 611a, 709a);
wherein said method comprises a step of the tension reducing device deflecting the one or more conductor wires (39a, 39b, 39c, 40) in such a manner as to arrange the one or more conductor wires (39a, 39b, 39c, 40) of the connector (19) around the deflection wall (215, 313a, 413a, 415a, 511a, 611a, 709a) that extends along an axis (P, P', T) that is out of alignment with the longitudinal axis (C) of the tension reducing device (200, 300, 400, 500, 600, 700, 800, 900);
in such a manner that when the at least one conductor wire (39a, 39b, 39c, 40) is stressed in tension, the at least one conductor wire (39a, 39b, 39c, 40) is blocked against the deflection wall (215, 313a, 413a, 415a, 511a, 611a, 709a) of the tension reducing device (200, 300, 400, 500, 600, 700, 800, 900).
